# EUROPEAN PATENT APPLICATION

(11) **EP 1 587 016 A1**
(43) Date of publication of application: **19.10.2005**
(21) Application number: 03774129.5
(22) Date of filing: 21.11.2003
(51) Int. Cl.: G06F 19/00, C12N 15/09, C12Q 1/68

(54) **METHOD OF IDENTIFYING DISEASE-SENSITIVITY GENE AND PROGRAM AND SYSTEM TO BE USED THEREFOR**

(30) Priority: 22.11.2002 JP 2002339901
(71) Applicant: Itakura, Mitsuo, Tokushima-shi, Tokushima 770-0042 (JP)
(72) Inventor: ITAKURA, Mitsuo, Tokushima-shi, Tokushima 770-0042 (JP); KATO, Hitoshi, Tokushima-shi, Tokushima 770-0042 (JP); KATASHIMA, Rumi, Tokushima-shi, Tokushima 770-0042 (JP); SHINOHARA, Syuichi, c/o Fujitsu Limited, Kawasaki-shi, Kanagawa 211-8588 (JP); NOMURA, Kyoko, c/o Fujitsu Limited, Kawasaki-shi, Kanagawa 211-8588 (JP)
(74) Representative: Forstmeyer, Dietmar
(86) International application number: PCT/JP2003/014888
(87) International publication number: WO 2004/049234

(57) **Abstract**

The object of this invention is to provide a method for efficiently identifying a disease susceptibility gene and, in particular, a disease susceptibility gene of a disease such as a multifactorial disease where a large number of genes are involved.

This invention relates to a method for identifying a disease susceptibility gene including selecting a plurality of SNP markers so as not to be unevenly distributed throughout a candidate region for the disease susceptibility gene, comparing by statistical processing a healthy control group and a diseased group with respect to the SNP markers selected, choosing SNP markers that exhibit a significant difference, comparing by statistical processing a healthy control group and a diseased group that are different from the groups above, specifying a SNP marker that exhibits a significant difference as a disease susceptibility SNP marker, and identifying a gene by subjecting the disease susceptibility SNP marker to a linkage disequilibrium analysis and locating a region, within the target candidate region, in which linkage disequilibrium is observed and which contains the disease susceptibility SNP marker, and a program and a system therefor.

SEQUENCE LISTING

<110> ITAKURA, Mitsuo
Applied Biosystems Japan Ltd.
FUJITSU LIMITED
Yamanouchi Pharmaceutical Co., Ltd.

<120> Methods for Identifying Susceptibility Genes to
Diseases, and Programs and Systems therefor

<130> 2111AB3206

<140>
<141>

<160> 14

<170> PatentIn Ver. 2.1

<210> 1
<211> 25
<212> DNA
<213> Homo sapiens

<400> 1
agtagaatga cccctaagct ttttt 25

<210> 2
<211> 25
<212> DNA
<213> Homo sapiens

<400> 2
gaaagctgat ggaataattc cctgt 25

<210> 3
<211> 25
<212> DNA
<213> Homo sapiens

<400> 3
gtaccaaggg agggctgcaa cagcc 25

<210> 4
<211> 25
<212> DNA
<213> Homo sapiens

<400> 4
gcatccattc aacaccactc ccaag 25

<210> 5
<211> 25
<212> DNA
<213> Homo sapiens

<400> 5
gcatttgtta attccagtac tagaa                    25


<210> 6
<211> 25
<212> DNA
<213> Homo sapiens

<400> 6
gtctgcattg caaaaccaga aatgt                    25


<210> 7
<211> 3244
<212> DNA
<213> Homo sapiens

<220>
<221> CDS
<222> (28)..(1293)

<400> 7
taatagaaca cttcacctga acctaaa atg gtg agc gag agt cac cat gag gcc 54
ctg gca gcc ccg cct gtc acc act gtc gcg act gtt ctg cca agc aat   102
gcc aca gag cca gcc agt cct gga gaa gga aag gaa gat gca ttt tct   150
aag ctg aag gag aag ttt atg aat gag ttg cat aaa att cca ttg cca   198
ccg tgg gcc tta att gca ata gcc ata gtc gca gtc ctt tta gtc ctg   246
acc tgc tgc ttt tgt atc tgt aag aaa tgt ttg ttc aaa aag aaa aac   294
aag aag aag gga aag gaa aaa gga ggg aag aat gcc att aac atg aaa   342
gat gta aaa gac tta ggg aag acg atg aaa gat cag gcc ctc aag gat   390
gat gat gct gaa act gga ttg aca gat gga gaa gaa aaa gaa gaa ccc   438
aaa gaa gag gag aaa ctg gga aaa ctt cag tat tca ctg gat tat gat   486
ttc caa aat aac cag ctg ctg gta ggg atc att cag gct gct gaa ctg   534
ccc gcc ttg gac atg ggg ggc aca tct gat cct tac gtg aaa gtg ttt   582
ctg cta cct gat aag aag aag aaa ttt gag aca aaa gtc cac cga aaa   630
acc ctt aat cct gtc ttc aat gag caa ttt act ttc aag gta cca tac   678
tcg gaa ttg ggt ggc aaa acc cta gtg atg gct gta tat gat ttt gat   726
cgt ttc tct aag cat gac atc att gga gaa ttt aaa gtc cct atg aac   774
aca gtg gat ttt ggc cat gta act gag gaa tgg cgt gac ctg caa agt   822
gct gag aag gaa gag caa gag aaa ttg ggt gat atc tgc ttc tcc ctt   870
cgc tac gta cct act gct ggt aag ctg act gtt gtc att ctg gag gca   918
aag aac ctg aag aag atg gat gtg ggt ggc tta tcc gat cct tat gtg   966
aag att cat ctg atg cag aat ggt aag agg ctg aag aag aaa aag aca   1014
aca att aaa aag aac aca ctt aac ccc tac tac aat gag tca ttc agc   1062
ttt gaa gta cct ttt gaa caa atc cag aaa gtg cag gtg gtg gta act   1110
gtt ttg gac tat gac aag att ggc aag aac gat gcc atc ggc aaa gtc   1158
ttt gtg ggc tac aac agc acc ggc gcg gag ctg cga cac tgg tca gac 1206
atg ctg gcc aac ccc agg cga cct att gcc cag tgg cac acc ctg cag 1254
gta gag gag gaa gtt gat gcc atg ctg gcc gtc aag aag taaaggaaag 1303
aagaagcctt tctgcatttg cccatatagt gctctttagc cagtatctgt aaatacctca 1363
gtaatatggg tcctttcatt tttccagcca tgcattccta acacaattca gtggtacttg 1423
gaatcctgtt ttaatttgca caaatttaaa tgtagagagc ccctaagtcc ttcatcatac 1483
cactgccctc caaatctact cttcttttaa gcaatatgat gtgtagatag agcatgaatg 1543
aaattattta ttgtatcaca ctgttgtata taccagtatg ctaaagattt atttctagtt 1603
tgtgtatttg tatgttgtaa gcgtttccta atctgtgtat atctagatgt ttttaataag 1663
atgttctatt ttaaactatg taaattgact gagatatagg agagctgata atatattata 1723
cggtaaatat agtatcgtct gcattccagc aaaaatatca actcgtaagg cactagtaca 1783
gttaaactga catcttaaag gacaacttaa acctgagctt tctattgaat catttgagta 1843
ccaagataaa cttacaccac atacttggtg ggtgaatcca attttgtaga attcctacac 1903
aggcaaaata gcatgatctg agcagcagca tccaggctga cctcaaggaa gcatagccac 1963
aaaacagaat agcacctgtc tgtacatatt tacaaagcta aaataatggc ttcactctta 2023
tatttgagga agcaactgaa caggagtcaa tgatttcata ttactgcata tagaataaca 2083
acaaggtgtt ccgtgtgtgt gtgtgtgtgt gtgtgtgtgt gtgtgtgtgt gtgtgtgcac 2143
atttgtttgg ggatggggga gaagaagcta aggggagaag tcaacattta tgaaatattg 2203
cctgactatt taaaaagaaa aaagtagctc tccattatca cctttataca aaatgtacat 2263
cctgtgaatt ctgttccaga tttcacacct acaataattc caaaaggttt gcacattaga 2323
gtttgtaaca aaatatttta ttatataaaa ccaggttaga aggaatgcag gatattttta 2383
acacaacaat ctgtgcttat tacacaaaat tactttgtgg taaacagaca gtattgtaat 2443
cccatcaaaa gatgaaagaa aaacaaaaac aaaaaccaac aacaattagc catagttctg 2503
aatgcacttc aattaagcca aaacagacag ctagtgatct ttttatatgc tctttttact 2563
taagttttaa tttgtccttt aaaaaaaggt gaaacaaacc aagaacaagt tctagaaaac 2623
tgaagcaacc tcttatgtat actagatgct tgatttagga ggagttttta aacgttttca 2683
atgttattat gtagtaaatg acactattat gaagctacta gtcattccat aagagtctta 2743
aaggactgct ctgtgtacac tgtgactgcc gtgtgtgctt agacccgtag tttcctcagt 2803
ggatagcact caatttattc cgtagtgata ttgtaacaat actgccattc ccttctactg 2863
cactgcccaa ggtgtgtgta gcacaaacag ttctcattac aaaggaccaa ttcagaactg 2923
aaaagctatg cataggacaa ggaagataca tagaatgggg tggaacacag cattttgtca 2983
agcactgtgc aatattccat atttttcccc actatggtag acaaccattt cgtggaaggg 3043
cagcctatta tcccacactg catctagcct tttgtcccat tcacttctgt gatccatttt 3103
aatttccagg ccacaagaca gtagtgatgc tctgaaatga aagtttgtct tcacaaatat 3163
caaaacaaaa tggaggaaaa ctaagcattg gcctcatgtt cagtcttcag gatatcacac 3223
cacgtctttt caaaaactaa a 3244

<210> 8
<211> 422
<212> PRT
<213> Homo sapiens

<400> 8

<210> 9
<211> 2210
<212> DNA
<213> Homo sapiens

<220>
<221> CDS
<222> (591)..(1184)

<400> 9
agccgcggcc tcaactaaaa gtggccattg acctttcaag ctttcgagca gtgatgcaat 60

agaatagtat ttcaaagaaa aatgcttatc gaaattttgg atccggtttt cccgtgattg 120

ttaagggttt cttttaaaaa gtaggtcaca tttcaagtag gtcatatttc gggggcgggt 180

gcgcagacaa ggagatgagt ttccactaag gccagggggc ctccaacggg gttggaggtg 240

agaatcccag gtagggtaga ggtgccgaga tccttccgaa tcccagccct ggggcgtcag 300

ccctgcaggg aatggcagag acactctccg gactgaggga accgaggcca gtcaccaagc 360

cccttccggg cgcgcaggcg atcagtgggt gaccgcggct gcgagggact ttgtcatccg 420

tcctccagga tctggggaga aagagcccca tcccttctct ctctgccacc atttcggaca 480

ccccgcaggg actcgttttg ggattcgcac tgacttcaag gaaggacgcg aacccttctc 540

tgaccccagc tcgggcggcc acctgtcttt gccgcggtga cccttctctc atg acc 596
Met Thr
1

ctg cgg tgc ctt gag ccc tcc ggg aat ggc ggg gaa ggg acg cgg agc 644
Leu Arg Cys Leu Glu Pro Ser Gly Asn Gly Gly Glu Gly Thr Arg Ser
        5                   10                  15

cag tgg ggg acc gcg ggg tcg gcg gag gag cca tcc ccg cag gcg gcg 692
Gln Trp Gly Thr Ala Gly Ser Ala Glu Glu Pro Ser Pro Gln Ala Ala
    20                  25                  30

cgt ctg gcg aag gcc ctg cgg gag ctc ggt cag aca gga tgg tac tgg 740
Arg Leu Ala Lys Ala Leu Arg Glu Leu Gly Gln Thr Gly Trp Tyr Trp
35                  40                  45                  50

gga agt atg act gtt aat gaa gcc aaa gag aaa tta aaa gag gca cca 788
Gly Ser Met Thr Val Asn Glu Ala Lys Glu Lys Leu Lys Glu Ala Pro
                55                  60                  65

gaa gga act ttc ttg att aga gat agc tcg cat tca gac tac cta cta 836
Glu Gly Thr Phe Leu Ile Arg Asp Ser Ser His Ser Asp Tyr Leu Leu
            70                  75                  80

aca ata tct gtt aaa aca tca gct gga cca act aat ctt cga atc gaa 884
Thr Ile Ser Val Lys Thr Ser Ala Gly Pro Thr Asn Leu Arg Ile Glu
        85                  90                  95

tac caa gac gga aaa ttc aga ttg gac tct atc ata tgt gtc aaa tcc 932
Tyr Gln Asp Gly Lys Phe Arg Leu Asp Ser Ile Ile Cys Val Lys Ser
    100                 105                 110

aag ctt aaa caa ttt gac agt gtg gtt cat ctg atc gac tac tat gtt 980
Lys Leu Lys Gln Phe Asp Ser Val Val His Leu Ile Asp Tyr Tyr Val
115                 120                 125                 130

cag atg tgc aag gat aag cgg aca ggt cca gaa gcc ccc cgg aac ggc 1028
Gln Met Cys Lys Asp Lys Arg Thr Gly Pro Glu Ala Pro Arg Asn Gly
                135                 140                 145

act gtt cac ctt tat ctg acc aaa ccg ctc tac acg tca gca cca tct 1076
Thr Val His Leu Tyr Leu Thr Lys Pro Leu Tyr Thr Ser Ala Pro Ser
            150                 155                 160

ctg cag cat ctc tgt agg ctc acc att aac aaa tgt acc ggt gcc atc 1124
Leu Gln His Leu Cys Arg Leu Thr Ile Asn Lys Cys Thr Gly Ala Ile
        165                 170                 175

tgg gga ctg cct tta cca aca aga cta aaa gat tac ttg gaa gaa tat 1172
Trp Gly Leu Pro Leu Pro Thr Arg Leu Lys Asp Tyr Leu Glu Glu Tyr
    180                 185                 190

aaa ttc cag gta taaatgtttc tcttttttta aacatgtctc acatagagta 1224
Lys Phe Gln Val
195

tctccgaatg cagctatgta aaagagaacc aaaacttgag tgctctggat aactatatgg 1284

aatgctttct aagaacagct gaagctaatc taatttaaat ttaacagctt gaagaggtag 1344

ctaggtgttt aaagttcctc cagatacttt tacctgagtg atgcttccct tcctaaggct 1404

gaccaagacc tgttgatcct tttagattaa aaataaaatg tcgcatgtaa aggctgaagt 1464

cgcgttttat cagaatgcct tgccttctta ggttcttttc cattatgtca aaggtccagg 1524

ctccagtagg agagaaagaa ctcctcatag gaatactgaa gaagtgggaa ggaaccaagc 1584

tgacacaggc ctcactgcaa tttgatatgc ctgctgatca gagtctcttg ggcattttat 1644

attttgcatt ctgatgtacc taggagtttt gttaaacaga tgatgtatgt gagtatttat 1704

cccattttat gcaattaacc aaatcaacca aaaaaagtga ccatgaagtc ctgtatttgt 1764

ctttttacta catgtaggaa ctctcatgtg aatgagtact gtagtaatcc attctatggg 1824

agccttattt cagaaatatt tcaaactggt gcaaatggaa aagactttct cttttccttt 1884

aaagctaaag acaagaatat catgctatac aggtgcaact caatccccgt taataaaaac 1944

caatgtaggt ataggcattc taccctttga aatagctgtg tcccaacctg ttgccattga 2004

ttttttggaa atggctttag aaatatccaa gttgtccttg aattgtctaa ccatggacat 2064

aaacagttgt ctcccttcta ctgtgtagaa tactttgact taattttctt ccagatacag 2124

ggggatacct gcctgttttt caaagtgttt atttactgct gttactattt gattagaatg 2184

tattaaataa aaaaaacctg atttct 2210

<210> 10
<211> 198
<212> PRT
<213> Homo sapiens

<400> 10
Met Thr Leu Arg Cys Leu Glu Pro Ser Gly Asn Gly Gly Glu Gly Thr
1               5                   10                  15

Arg Ser Gln Trp Gly Thr Ala Gly Ser Ala Glu Glu Pro Ser Pro Gln
            20                  25                  30

Ala Ala Arg Leu Ala Lys Ala Leu Arg Glu Leu Gly Gln Thr Gly Trp

35          40          45

Tyr Trp Gly Ser Met Thr Val Asn Glu Ala Lys Glu Lys Leu Lys Glu
    50          55          60

Ala Pro Glu Gly Thr Phe Leu Ile Arg Asp Ser Ser His Ser Asp Tyr
65          70          75          80

Leu Leu Thr Ile Ser Val Lys Thr Ser Ala Gly Pro Thr Asn Leu Arg
            85          90          95

Ile Glu Tyr Gln Asp Gly Lys Phe Arg Leu Asp Ser Ile Ile Cys Val
        100         105         110

Lys Ser Lys Leu Lys Gln Phe Asp Ser Val Val His Leu Ile Asp Tyr
    115         120         125

Tyr Val Gln Met Cys Lys Asp Lys Arg Thr Gly Pro Glu Ala Pro Arg
  130         135         140

Asn Gly Thr Val His Leu Tyr Leu Thr Lys Pro Leu Tyr Thr Ser Ala
145         150         155         160

Pro Ser Leu Gln His Leu Cys Arg Leu Thr Ile Asn Lys Cys Thr Gly
            165         170         175

Ala Ile Trp Gly Leu Pro Leu Pro Thr Arg Leu Lys Asp Tyr Leu Glu
        180         185         190

Glu Tyr Lys Phe Gln Val
    195

<210> 11
<211> 24
<212> DNA
<213> Artificial Sequence

<220>
<223> Description of Artificial Sequence: Forward primer
   for typing SNP260

<400> 11
gcaaagtaga atgaccccta agct                          24

<210> 12
<211> 25
<212> DNA
<213> Artificial Sequence

<220>
<223> Description of Artificial Sequence: Reverse primer
   for typing SNP260

<400> 12
gcataaagta ggctgacagg gaatt                         25

<210> 13
<211> 15
<212> DNA
<213> Artificial Sequence

<220>
<223> Description of Artificial Sequence: VIC probe for
   typing SNP260

<400> 13
tcagctttcg aaaaa                                        15


<210> 14
<211> 16
<212> DNA
<213> Artificial Sequence

<220>
<223> Description of Artificial Sequence: FAM probe for
   typing SNP260

<400> 14
atcagctttc caaaaa                                       16

## Description

### [Detailed Description of the Invention]

### [Field of the Invention]

The present invention relates to a method for identifying disease susceptibility genes using SNPs (Single Nucleotide Polymorphisms), and a program and a system therefor.

### [Prior Art]

Genome analysis is currently advancing, and study of the association between diseases and genes is underway worldwide. Identifying a gene that is involved in a disease is highly significant from the viewpoint of treatment and prevention of the disease.

In this respect, with regard to disease susceptibility genes that are involved in a disease caused by a single gene or a disease involving noticeable gene mutation, identification thereof can be comparatively easy by, for example, comparing gene expression, etc. between healthy controls and diseased individuals. However, in the case where a plurality of genes are involved in a disease, identification of such genes is difficult (see. Non-patent document 1).

"Association Analysis by the Candidate Gene Approach", in which a candidate susceptibility gene is set from functional aspects, expression information, etc., and whether or not it is associated with the disease is examined, the "Positional Candidate Method", in which a candidate gene is selected by adding to the above information positional information obtained by linkage analysis, etc. are conventionally known (see. Non-patent document 2 and Non-patent document 3). However, the method of candidate selection depends on previously known functional information or expression information, and in order to employ these methods it is necessary to obtain such information for a plurality of susceptibility genes. It can therefore be said that there is almost no possibility that these methods can identify a disease susceptibility gene related to an unknown new signal pathway or a disease susceptibility gene with unknown function.

On the other hand, with regard to genetic mutations that cause diseases, much attention has been paid to genetic polymorphisms and, in particular, SNPs (Single Nucleotide Polymorphisms) have been studied with intense interest in terms of the relationship between polymorphism and disease. Information about SNPs is stored in several databases and, for example, about 2.7 million unique SNPs are registered with the American dbSNP database (SNP database created by NCBI (the National Center for Biotechnology Information), URL: http://www.ncbi.nlm.nih.gov/SNP/index.html). In Japan, the Institute of Medical Science of the University of Tokyo and the Japan Science and Technology Corporation have published the results of frequency analyses by focusing on genomic regions containing genes, detecting about 190,000 SNPs using genomic DNA from 24 Japanese individuals, and 339 (678 alleles) to 752 (1504 alleles) Japanese individuals have been subjected to an analysis in relation to about 78,000 SNPs thereamong (see. Non-patent document 4 and Non-patent document 5). Research into clarifying the relationship between SNPs and disease has been progressing on the basis of such findings.

In recent years, attention has been paid to "common diseases", and identification of their disease susceptibility genes has been carried out. Typical examples thereof include human type 2 diabetes; this disease is a multifactorial disease that develops as a result of involvement of both genetic factors, such as a large number of related genes, and environmental factors, and a large number of genetic studies thereof have been reported (see. Non-patent document 6, Non-patent document 7 and Non-patent document 8).

In particular, it has become clear from linkage analysis that there is a type 2 diabetes susceptibility region that is repeatedly linked beyond ethnic groups and races, and it has been surmised that it is highly possible that a disease susceptibility gene that is common to each race and relates to the development of type 2 diabetes, that is, a common original gene (a disease susceptibility gene that satisfies a Common Disease - Common Variant - Common Origin hypothesis) is present in the above region (see. Non-patent document 2).

However, the disease susceptibility region obtained by linkage analysis spreads over a wide range; a direct relationship between the "linkage analysis" and "susceptibility SNPs of a disease susceptibility gene" that causes the linkage is shown in only one example, that of calpain-10 (see. Non-patent document 9 and Non-patent document 10), and other reports of the linkage analysis only indicate "a widespread disease susceptibility region".

Although "common diseases" such as type 2 diabetes are important diseases in terms of the number of diseased individuals, the reason that disease susceptibility genes cannot be found is mainly because, as hereinbefore described, they are multifactorial diseases; a large number of genes are presumed to be involved, and it is therefore difficult to identify the disease susceptibility genes.

Consequently, there has been a desire for the development of a novel method for identifying a disease susceptibility gene and, in particular, a method for identifying disease susceptibility genes for a disease such as a "common disease" where a large number of genes are presumed to be involved.

### [Non-patent document 1]

"Diabetes Frontier", Medical Review, February 2002, Vol. 12, No. 1, p. 44-49.

### [Non-patent document 2]

"Clinical Genetics, October 2001, Vol. 60, No. 4, p. 243-54.

### [Non-patent document 3]

"Best Practice & Research Clinical Endocrinology & Metabolism" July 2001, Vol. 15, No. 3, p. 293-308.

### [Non-patent document 4]

"JSNP Database", The University of Tokyo Institute of Medical Science and Japan Science and Technology Corporation, [online], October 21st, 2002, [Searched on November 12th, 2002], Internet <URL: http://snp.ims.u-tokyo.ac.jp/release_notes.html>

### [Non-patent document 5]

"Nucleic Acids Research", January 2002, Vol. 30, No. 1, p. 158-162.

### [Non-patent document 6]

"American Journal of Physiology - Endocrinology and Metabolism", August 2002, Vol. 283, No. 2, E217-25.

### [Non-patent document 7]

"Clinical Genetics", October 2001, Vol. 60, No. 4, p. 243-54.

### [Non-patent document 8]

"Saishin Igaku", Saishin Igaku, March 2000, Vol. 55, No. 3, p. 316-322.

### [Non-patent document 9]

"Nature Genetics", (US), Nature America Inc., October 2000, Vol. 26, No. 2, p. 163-175.

### [Non-patent document 10]

"The Journal of Saitama Medical School", January 2002, Vol. 29, No. 1, p. 77-84.

### [Disclosure of the Invention]

It is therefore an object of the present invention to solve the above-mentioned problems of the art, and provide a method for efficiently identifying a disease susceptibility gene and, in particular, a disease susceptibility gene for a disease such as a multifactorial disease where a large number of genes are involved.

As a result of an intensive investigation by the present inventors in view of the above-mentioned object while focusing attention on the association between SNPs and disease, it has been found that SNPs that are in linkage disequilibrium with disease susceptibility genes can be selected effectively by screening SNPs within a fixed genomic region at certain intervals and subjecting these SNPs to two-step screening, and the present invention has thus been accomplished.

That is, the present invention is a method for identifying disease susceptibility genes using SNP markers, the method comprising;
(1) a step in which a plurality of SNP markers are selected from within a candidate region for the disease susceptibility gene using samples from healthy control(s), the SNP markers not being unevenly distributed throughout the candidate region;
(2) a step in which, for the SNP markers selected in step (1), a comparison is made by statistical processing between a healthy control group and a diseased group, and SNP markers that exhibit a significant difference are chosen;
(3) a step in which, for the SNP markers chosen in step (2), a comparison is made by statistical processing between a healthy control group and a diseased group that are different from those of step (2), and a SNP marker that exhibits a significant difference is specified as a disease susceptibility SNP marker; and
(4) a step in which a gene is identified by subjecting the disease susceptibility SNP marker to a linkage disequilibrium analysis and locating a region, within the target candidate region, in which linkage disequilibrium is observed and which contains the disease susceptibility SNP marker.

Furthermore, the present invention relates to the above-mentioned method in which the selection of SNP markers in step (1) is carried out so that the marker density is at least 1 SNP per 10 kb in the target candidate region.

Moreover, the present invention relates to the above-mentioned method in which the selection of SNP markers in step (1) is carried out so that the interval between adjacent SNP markers is at least 5 kb.

Furthermore, the present invention relates to the above-mentioned method in which the selection of SNP markers in step (1) is carried out on the basis of gene frequency.

Moreover, the present invention relates to the above-mentioned method in which the basis of gene frequency is that the minor allele gene frequency is at least 10%.

Furthermore, the present invention relates to the above-mentioned method in which the basis of gene frequency is that the minor allele gene frequency is at least 15%.

Moreover, the present invention relates to the above-mentioned method in which the selected SNP markers are evaluated by repeating step (1) using samples from healthy control(s) that are different from those used in step (1).

Furthermore, the present invention relates to the above-mentioned method wherein the evaluation is carried out by a Hardy-Weinberg equilibrium test.

Moreover, the present invention relates to the above-mentioned method wherein the statistical processing in step (2) is an association analysis.

Furthermore, the present invention relates to the above-mentioned method wherein the statistical processing in step (3) is an association analysis.

Moreover, the present invention relates to the above-mentioned method wherein the significance level in the comparison in step (3) is lower than the significance level in the comparison in step (2).

Furthermore, the present invention relates to the above-mentioned method wherein the association analysis in step (2) is a χ² test for gene frequency, and a SNP marker that exhibits a significant difference with a significance level of α ≤ 0.10 is chosen, and the association analysis in step (3) is carried out by a χ² test for gene frequency, and a SNP marker that exhibits a significant difference with a significance level of α < 0.10 is chosen.

Moreover, the present invention relates to the above-mentioned method in which the linkage disequilibrium analysis for the disease susceptibility SNP marker in step (4) is carried out for the SNP markers that are selected in step (1) and the disease susceptibility SNP marker.

Furthermore, the present invention relates to the above-mentioned method in which the number of SNP markers, including the disease susceptibility SNP marker, that are subjected to the linkage disequilibrium analysis is at least 4.

Moreover, the present invention relates to a disease susceptibility SNP marker that is located by means of the ability to exhibit a significant difference when comparing by statistical processing, using samples from healthy control(s), a healthy control group and a diseased group with respect to a plurality of SNP markers that are selected so as not to be unevenly distributed throughout a candidate region for a disease susceptibility gene, choosing SNP markers that exhibit a significant difference, and further comparing by statistical processing a different healthy control group and a different diseased group with respect to the chosen SNP markers.

Furthermore, the present invention relates to a disease diagnosis marker containing one or more polynucleotides chosen from the group consisting of human genome polynucleotides having a length that can be specifically recognized on the human genome, the polynucleotides containing each SNP marker among one or more SNP markers that are present in a region where linkage disequilibrium is observed within a target candidate region in a linkage disequilibrium analysis with respect to the above-mentioned disease susceptibility marker, the region where linkage disequilibrium is observed containing the disease susceptibility SNP marker.

Moreover, the present invention relates to a diabetes susceptibility diagnosis marker containing one or more polynucleotides chosen from the group consisting of
a polynucleotide in which a base sandwiched between a sequence represented by SEQ ID NO:1 and a sequence represented by SEQ ID NO:2 within a genomic sequence is C or G,
a polynucleotide in which a base sandwiched between a sequence represented by SEQ ID NO:3 and a sequence represented by SEQ ID NO:4 within the genomic sequence is A or G, and
a polynucleotide in which a base sandwiched between a sequence represented by SEQ ID NO:5 and a sequence represented by SEQ ID NO:6 within the genomic sequence is C or T.

Furthermore, the present invention is a diabetes susceptibility diagnosis method comprising:
(1) a step in which genomic DNA is extracted from a sample, and
(2) a step including, with regard the sequence of the extracted genomic DNA, one or more chosen from the group consisting of
detecting a base sandwiched between a sequence represented by SEQ ID NO:1 and a sequence represented by SEQ ID NO: 2 and, in particular, detecting whether it is C or G,
detecting a base sandwiched between a sequence represented by SEQ ID NO:3 and a sequence represented by SEQ ID NO:4 and, in particular, detecting whether it is A or G, and
detecting a base sandwiched between a sequence represented by SEQ ID NO:5 and a sequence represented by SEQ ID NO: 6 and, in particular, detecting whether it is C or T.

Moreover, the present invention relates to a program for allowing a computer to execute
(1) a step in which, based on data for samples from healthy control (s) including base data for a healthy control group, the minor allele frequency is calculated for each SNP, SNPs that have a calculated value of at least a set selection value are selected, and these SNPs are output,
(2) a step in which base data, corresponding to the SNPs output in step (1), for a diseased group are input, a comparison is made by statistical processing between the base data for the healthy control group and the base data for the diseased group, and SNPs that exhibit a significant difference are output as chosen SNP markers, and
(3) a step in which base data, corresponding to the SNP markers output in step (2), for a healthy control group and a diseased group that are different from those used in step (2) are input, a comparison is made by statistical processing between the base data for the healthy control group and the base data for the diseased group, and it is determined that a SNP marker that exhibits a significant difference is a disease susceptibility SNP marker.

Furthermore, the present invention relates to a disease susceptibility gene identification system for identifying a disease susceptibility gene, the system comprising:
(1) means for calculating, based on data for samples from healthy control(s) including base data for a healthy control group, the minor allele frequency for each SNP, selecting SNPs that have a calculated value of at least a set selection value, and outputting these SNPs,
(2) means for inputting base data for a diseased group corresponding to the SNPs output by means (1), comparing by statistical processing the base data for the healthy control group and the base data for the diseased group, and outputting those that exhibit a significant difference as chosen SNP markers,
(3) means for inputting base data, corresponding to the SNP markers output by means (2), of a healthy control group and a diseased group that are different from those used for means (2), comparing by statistical processing the base data for the healthy control group and the base data for the diseased group, and determining that one that exhibits a significant difference is a disease susceptibility SNP marker, and
(4) means for subjecting the disease susceptibility SNP marker to a linkage disequilibrium analysis and determining within a target candidate region a region in which linkage disequilibrium can be observed and which contains the disease susceptibility SNP marker.

In accordance with the above-mentioned arrangements, the present invention allows highly reliable SNP markers to be selected, at reasonable intervals not being unevenly distributed throughout a target candidate region, where a disease susceptibility gene is thought to be present. Setting SNP markers in this way can ease the experimental burden, etc. since the number of SNP markers to be analyzed can be comparatively small. Furthermore, a disease susceptibility gene in any part of the target candidate region can be identified efficiently by a linkage disequilibrium analysis. Moreover, choosing SNPs having a comparatively high minor allele frequency, etc. makes it possible to set SNP markers, specify a disease susceptibility marker, identify a disease susceptibility gene, etc. even when using a population having a comparatively small sample size.

Although in the disease susceptibility gene identification method of the present invention it is possible to use the entire genomic region as a target for identifying a disease susceptibility gene, it is effective to utilize conventional findings about a genomic region associated with a disease obtained by a linkage analysis, etc. and use this genomic region as a region containing a disease susceptibility gene (or part thereof). In the present specification, such a region is called a target candidate region (candidate region for the disease susceptibility gene).

The "disease susceptibility gene" referred to in the present specification means a plurality of genes that determine a predisposition to easily contract a multigenic disease.

Furthermore, the "gene frequency" referred to in the present specification means, with respect to one gene locus, the proportion of an allele in the total number of genes present in a population.

Moreover, the "linkage disequilibrium analysis" referred to in the present specification means an analysis of the extent of linkage disequilibrium in a genomic region. In examples, the linkage disequilibrium analysis was carried out by calculating a linkage disequilibrium coefficient |D'|, which represents the extent of linkage disequilibrium between two markers, from typing data for 164 unrelated healthy controls.

Furthermore, the "minor allele" referred to in the present specification means an allele having a low gene frequency when two alleles are present at one gene locus.

Moreover, the "polymorphism" referred to in the present specification refers to alleles when there are two or more genetically determined alleles. The "single nucleotide polymorphism" referred to means polymorphism caused by change of a single nucleotide. The polymorphisms are present with a frequency higher than 1% of a chosen population, and preferably with a frequency of at least 10%.

Furthermore, the "linkage disequilibrium" referred to in the present specification means a relationship in which an allele occurs together with a specific allele in the vicinity at a frequency higher than that expected by chance for any combination of alleles in a population. For example, in the case where a gene locus X has alleles a and b (present with equal frequencies) and a gene locus Y in the vicinity has alleles c and d (present with equal frequencies), haplotype ac, which is a combination of different genetic polymorphisms, is expected to be present in the population with a frequency of 0.25. If haplotype ac is seen at more than this expected value, that is, if the specific genotype ac occurs more frequently, then it is said that the alleles ac are in linkage disequilibrium. The linkage disequilibrium is due to natural selection of a specific combination of alleles or an evolutionarily recent introduction thereof into the population, and can occur because linked alleles have not yet reached equilibrium with each other. The manner of linkage disequilibrium therefore varies among populations such as ethnic groups and races, and even if ac is in linkage disequilibrium in one population, ad can be in linkage disequilibrium in another population. Polymorphisms in linkage disequilibrium, even though the polymorphisms do not cause a disease, can be effective for detecting a susceptibility to the disease. For example, allele a of gene locus X is not a causative genetic element for a disease, but it may exhibit disease susceptibility by linkage disequilibrium with allele c of gene locus Y.

### [Brief Explanation of Drawings]

[FIG. 1] A list of linkage disequilibrium coefficients |D'| between two SNPs and a schematic diagram showing the relative positions of the SNPs.
[FIG. 2] A list of linkage disequilibrium coefficients |D'| between two SNPs and a schematic diagram showing the relative positions of the SNPs.
[FIG. 3] A diagram showing one mode of a table used in the program of the present invention.
[FIG. 4] A diagram showing one mode of a table used in the program of the present invention.
[FIG. 5] A diagram showing one mode of a table used in the program of the present invention.
[FIG. 6] A diagram showing one mode of a table used in the program of the present invention.
[FIG. 7] A flow diagram showing one mode of the program of the present invention.

### [Best Embodiment of the Invention]

In the method of the present invention, SNP markers that are typed using samples from healthy control(s) for a target candidate region are selected. The "samples" referred to in the present specification are not particularly limited as long as they contain genomic DNA. For example, bodily fluids such as blood including peripheral blood, saliva, and sweat, somatic cells, tissue or organs containing same, etc. can be cited.

It is necessary for the healthy control group and the diseased group to be constituted of the same race as that for which a disease susceptibility gene is located; for example, in order to identify a disease susceptibility gene of Japanese people it is necessary for the control group to be constituted of healthy Japanese individuals. It is also possible to select SNP markers using various types of database for SNPs such as the US dbSNP database and the JSNP database by The University of Tokyo Institute of Medical Science and the Japan Science and Technology Corporation. However, in order to identify a gene precisely in a population that is genetically comparatively uniform such as a single race or a single ethnic group, it is preferable to subject samples from healthy control(s) belonging to a desired population to SNP typing with respect to a target candidate region.

With regard to SNP typing methods, methods known to a person skilled in the art, such as PCR-SSCP, PCR-RFLP, PCR-SSO, PCR-ASP, a direct sequence method, SNaPshot, dHPLC, a Sniper method, and a MALDI-TOF/MS method (see. e.g. "Genome Soyaku no Saizensen (Frontiers of Genome Medicine)", p44-p54, Ed. H. Nojima, Yodosha) can be used, but it is particularly effective to employ a SNP typing method that utilizes Assays-on-Demand® (manufactured by Applied Biosystems) and a TaqMan system.

In the method of the present invention, SNPs that are useful for identification of a gene are selected on the basis of the results of SNP typing, within a target candidate region, of samples from healthy control(s), and it is desirable to use gene frequency as an indicator for selection. Specifically, SNPs having a minor allele frequency of at least 10%, and preferably at least 15%, are selected. Use of SNPs having such a gene frequency makes it possible to select highly reliable SNP markers. Furthermore, when this gene frequency is high, good SNP markers can be selected with a comparatively small number of healthy control samples of on the order of 30 to 50 samples.

With regard to markers selected with a comparatively small number of samples, confirmation by comparing them with SNP markers selected using other healthy control samples and evaluation of the sampling validity and the assay validity by a Hardy-Weinberg equilibrium test allow sufficiently highly reliable SNP markers to be selected within the target candidate region.

When making a comparison with SNP markers selected using other healthy control samples, it should be confirmed that the selected SNP markers can also be selected for said other healthy control samples using the minor allele gene frequency as an indicator.

Furthermore, with regard to evaluation by the Hardy-Weinberg equilibrium test, the selected SNP markers are tested.

The Hardy-Weinberg equilibrium is well known in the field of genome statistics, and is used for evaluating errors in typing a genotype and for evaluating the validity of sampling. When two alleles (for example, C and T) are present, as in a SNP, etc., and the frequencies thereof in a population are p and q respectively (p + q = 1), the genotype frequencies of C/C homo, C/T hetero, and T/T homo are p², 2pq, and q² respectively (p² + 2pq + q² = 1). Although it is desirable for the Hardy-Weinberg equilibrium to be satisfied for a healthy control group, if the number of samples that have a statistically significant difference from the Hardy-Weinberg equilibrium is within an expected range at the significance level (typically, α = 0.01 to 0.05), then the selected SNP marker can be evaluated as being valid.

In the case where SNP markers are selected using gene frequency as an indicator, the SNP markers might be unevenly distributed in a specific narrow region. In this case, if all of the selected SNP markers are used for identifying a disease susceptibility gene, the experiment becomes complicated, and since SNPs in the vicinity of each other are often in a linkage disequilibrium state it is inefficient. In the method of the present invention, it is therefore preferable to select SNP markers from those roughly chosen with certain intervals. Ensuring that markers have certain intervals in this way so as to eliminate uneven distribution makes it possible to carry out an exhaustive association analysis throughout a target candidate region, thereby making it easy to identify a disease susceptibility gene. The distance between adjacent SNP markers thus selected is preferably at least 5 kb, and particularly preferably 5 kb to 10 kb. When this distance is too long, there is a possibility that in some regions the extent of linkage disequilibrium between SNP markers cannot be observed. When this distance is too short, many SNPs exhibit strong linkage disequilibrium between each other, thus increasing the amount of experimentation in a subsequent linkage disequilibrium analysis, which is inefficient.

When selecting SNP markers exhaustively throughout a target candidate region, in addition to the distance between the SNP markers, the manner in which the markers are spaced within the target candidate region can be expressed as a "marker density", that is, the number of markers per unit distance of the genome. The marker density of SNP markers selected in the method of the present invention is at least 0.5 SNPs per 10 kb of a genome, preferably at least 1 SNP, and particularly preferably 1 SNP to 2 SNPs.

When the marker density is too low, the distance between markers is too long, thus causing a possibility that in some areas the extent of linkage disequilibrium between SNP markers cannot be observed; and when the marker density is too high, the markers are selected too densely, thus increasing the amount of experimentation when identifying a gene, which is inefficient.

As hereinbefore described, the method of the present invention chooses SNPs associated with a disease by an association analysis between the disease and SNP markers selected at certain intervals throughout the target candidate region, the analysis involving a comparison of gene frequency between a healthy control group and a diseased group.

The association analysis is typically carried out by comparing the gene frequency of each of the SNP markers between a diseased group and a healthy control group and subjecting the difference in the gene frequency to a χ² test (see. Tokeigaku Nyumon - Kisotokeigaku I (Introduction to Statistics - Basic Statistics I), Ed. Department of Statistics, College of Arts and Sciences, The University of Tokyo, University of Tokyo Press) to find out whether or not the difference is statistically significant, but it can also be carried out in terms of the genotype frequency or the positive allele rate for each of the SNP markers. Furthermore, other than the χ² test, if it is possible to make a comparison between a diseased group and a healthy control group, that is, to test the association between genetic polymorphisms and phenotypic characteristics such as disease, there being a plurality of populations corresponding to the phenotypic characteristics, other known statistical processing can be employed.

In the method of the present invention, the association analysis is carried out for a different sampling group from the same respective population for each of the diseased group and the healthy control group. In a first association analysis, the detectability is enhanced by relaxing the significance level, thus detecting widely and including pseudopositives, and in a subsequent second association analysis disease susceptibility SNPs are detected and specified with a normal significance level for only the marker SNPs chosen in the first analysis. In the second analysis, targeting only the marker SNPs chosen in the first analysis can effectively suppress an increase in the pseudopositive rate due to multiple tests.

More specifically, it is carried out as follows (in the case of a χ² test for testing the independence of a disease from gene frequency).

Firstly, samples obtained from a diseased group D1 and a healthy control group H1 are each subjected to an association analysis (a χ² test for testing the independence of disease from gene frequency) for SNP markers selected within a target candidate region. As candidate SNPs for a disease susceptibility SNP, SNPs are chosen that exhibit a statistically significant difference in the gene frequency between D1 and H1, preferably SNPs that exhibit a significant difference at the significance level of α ≤ 0.10 (P < 0.10), and more preferably SNPs that exhibit a significant difference at the significance level of α ≤ 0.07 (P < 0.07).

With regard to the P value, the larger the P value, the lower the association with the disease, and the smaller the P value, the higher the association. However, in the case where candidate SNPs are chosen, if only those having a very small P value are selected, the detectability for a disease susceptibility SNP marker is greatly reduced, and the number of candidate SNPs might become too small. In this stage candidate SNPs are chosen so as to widely pick up marker SNPs that have a possibility of showing disease susceptibility, including those detected due to statistical pseudopositives.

An association analysis is then carried out for these chosen candidate SNPs using samples from a diseased group D2 and a healthy control group H2, which are different from the group D1 and the group H1 above. This association analysis is preferably carried out completely independently from the previous association analysis, but if the sample number of group D2 is larger than the sample number of group D1, it can be carried out without being completely independent. Furthermore, the sample number of group H2 is preferably larger than the sample number of group H1.

The association analysis (a χ² test for testing the independence of a disease from gene frequency) between groups D2 and H2 is carried out for the candidate SNPs obtained as a result of the association analysis for the groups D1 and H1. In this analysis, a SNP that exhibits a statistically significant difference in the gene frequency between D2 and H2 is specified as a disease susceptibility SNP marker.

The two association analyses are carried out independently in this way; firstly candidate SNPs are chosen under moderate conditions, and by then specifying disease susceptibility SNP markers under strict conditions, only the more highly reliable SNP markers whose association is detected by both of these two tests can be specified, and a group of SNPs that are detected as pseudopositives in the first analysis can be excluded in the second analysis.

The "moderate conditions" and the "strict conditions" referred to here mean relative conditions of the two association analyses that are carried out independently. For example, when the significance level α1 of the first association analysis and the significance level α2 of the second association analysis are compared, if α1 > α2, then the first association analysis is carried out under "moderate conditions" and the second association analysis is carried out under "strict conditions". In general, the smaller the significance level, for example, α = 1 x 10⁻³ or α = 1 x 10⁻⁴, the higher the reliability of the SNP markers chosen but, under conditions where α1 > α2, the purpose of the present invention can be achieved by setting the significance level of the first association analysis at α1 ≤ 0.10, preferably α1 ≤ 0.07, and more preferably α1 ≤ 0.05, and setting the significance level of the second association analysis at α2 < 0.10, preferably α2 ≤ 0.05, and more preferably α2 ≤ 0.01.

Furthermore, a disease susceptibility gene is identified by a linkage disequilibrium analysis using the specified disease susceptibility SNP marker.

The linkage disequilibrium analysis is a method known to a person skilled in the art and can be carried out by various types of conventional linkage disequilibrium analysis (see. e.g. "Post Genome Jidai no Iden Tokeigaku (Genetic Statistics in a Post Genome Era)", p.183 - p.201, Ed. N. Kamatani, Yodosha). When carrying out a linkage disequilibrium analysis, a commercial program such as, for example, the SNP disease association analysis software "SNPAlyze ver. 2.1" (manufactured by Dynacom Co., Ltd.) can be used. More specifically, an analysis can be carried out by calculating a linkage disequilibrium coefficient |D'| (pair-wise LD coefficient) by a linkage disequilibrium analysis employing an EM method.

SNP markers used in the linkage disequilibrium analysis are the specified disease susceptibility SNP marker and other markers in the vicinity thereof, but SNP markers previously selected within the target candidate region are preferably used. The number of markers used is at least 4 SNPs including the disease susceptibility SNP marker, preferably at least 20 SNPs, and particularly preferably at least 32 SNPs, and a group of SNP markers containing the above is used.

The number of markers in the group of SNP markers can be changed as appropriate according to the size of a region forming a haplotype block (linkage disequilibrium block) associated with a disease susceptibility gene that is to be identified. In the case where the end of the block can be predicted, about 6 SNPs having the block therebetween can be used. Furthermore, it is also possible to first carry out a linkage disequilibrium analysis for a total of 11 SNPs, that is, 5 SNPs on each side of the disease susceptibility SNP marker, and the number of markers to be analyzed can be increased as necessary.

As a result of the linkage disequilibrium analysis, a region where SNPs are linked to each other within the target candidate region (a haplotype block containing a group of SNP markers where strong linkage disequilibrium with each other is observed) is located. Locating a haplotype block can be carried out appropriately by a person skilled in the art based on the extent of the linkage disequilibrium, and can be carried out in accordance with, for example, a report by Gabriel et al. (Gabriel SB et al., Science 296 (5576): 2225-9 (2002)). That is, the "haplotype block" is defined as a region where strong linkage disequilibrium is present within the confines of a genome in which almost no historical recombination is observed; the "strong linkage disequilibrium" referred to here means a state in which the upper limit of the 95% confidence interval of |D'| exceeds 0.98 and the lower limit of the 95% confidence interval thereof is more than 0.7, and "strong proof of historical recombination" means a state in which the upper limit of the 95% confidence interval of |D'| is less than 0.9.

In particular, in the present specification, a linkage disequilibrium coefficient |D'| is calculated for all combinations of two SNPs among the selected SNP markers; combinations that exhibit a |D'| of >0.9 are chosen, and among these a series of regions containing a region sandwiched by the two SNPs that are the farthest from each other is defined as a haplotype block, 3 SNPs in series outside the haplotype block exhibiting a |D'| of at most 0.9 in combinations with any SNP within the haplotype block.

Once the haplotype block is located, a gene present in the haplotype block in question can be located utilizing, for example, a genome database for that region. Even in the case where no database is utilized, a base sequence in the vicinity of a SNP marker present in the haplotype block region can be determined by a standard method, and a gene can be located from the base sequence.

The present inventors have successfully identified three human type 2 diabetes susceptibility genes by the above-mentioned method for identifying a disease-related gene.

Furthermore, the present invention includes a disease diagnosis marker containing one or more polynucleotides chosen from the group consisting of polynucleotides having a length that can be specifically recognized on the human genome, the polynucleotides containing each SNP marker among one or more SNP markers that are present in a region (haplotype block) in which linkage disequilibrium can be observed within a target candidate region in a linkage disequilibrium analysis of a disease susceptibility marker, and which contains the disease susceptibility SNP marker. It is sufficient for the disease diagnosis marker to have a length that can be specifically recognized on the human genome and is, for example, at least 10 bases, and preferably at least 20 bases. It can therefore be, as necessary, 51 bases including the SNP marker in a middle (25 bases on each side), 201 bases (100 bases on each side), 601 bases (300 bases on each side), etc.

The present invention also includes a diabetes susceptibility diagnosis marker and a diagnostic method therefor.

An example of the diabetes susceptibility diagnosis marker is as follows:
a diabetes susceptibility diagnosis marker comprising
a polynucleotide in which the 843215th base in a sequence represented by NCBI Accession No. NT_019546 (version NT_019546.12), that is, a base (hereinafter referred to as SNP260) sandwiched between a sequence represented by SEQ ID NO:1 and a sequence represented by SEQ ID NO:2 in genomic sequence SYT1 is C or G,
a polynucleotide in which the 845590th base in a sequence represented by NCBI Accession No. NT_019546 (version NT_019546.12), that is, a base (hereinafter referred to as SNP262) sandwiched between a sequence represented by SEQ ID NO:3 and a sequence represented by SEQ ID NO:4 in the genomic sequence is A or G, or
a polynucleotide in which the 7573rd base in a sequence represented by NCBI Accession No. NT_009575 (version NT_009575.12), that is, a base (hereinafter referred to as SNP488) sandwiched between a sequence represented by SEQ ID NO:5 and a sequence represented by SEQ ID NO:6 in the genomic sequence is C or T.

The diabetes susceptibility diagnosis method is not particularly limited as long as it includes the following steps:
(1) a step in which genomic DNA is extracted from a sample, and
(2) a step including, for the extracted genomic DNA sequence, one or more chosen from the group consisting of detecting the 843215th base in a sequence represented by NCBI Accession No. NT_019546 (version NT_019546.12), that is, a base (SNP260) sandwiched between a sequence represented by SEQ ID NO:1 and a sequence represented by SEQ ID NO:2 in genomic sequence SYT1, and preferably detecting whether SNP260 is C or G,
detecting the 845590th base in a sequence represented by NCBI Accession No. NT_019546 (version NT_019546.12), that is, a base (SNP262) sandwiched between a sequence represented by SEQ ID NO:3 and a sequence represented by SEQ ID NO:4, and preferably detecting whether SNP262 is A or G, and
detecting the 7573rd base in a sequence represented by NCBI Accession No. NT_009575 (version NT_009575.12), that is, a base (SNP488) sandwiched between a sequence represented by SEQ ID NO:5 and a sequence represented by SEQ ID NO:6 in the genomic sequence, and preferably detecting whether SNP488 is C or T.

In the case where SNP260 is C, in the case where SNP262 is A, or in the case where SNP488 is C, a diagnosis of susceptibility to diabetes can be made, and in the case where SNP260 is G, in the case where SNP262 is G, or in the case where SNP488 is T, a diagnosis of no susceptibility to diabetes can be made.

Typing of SNP262 can employ a primer and probe of Product No. C 36615_10 of Assays-on-Demand®, and typing of SNP488 can employ a primer and probe of Product No. C 3188143_10 of Assays-on-Demand®.

Furthermore, typing of SNP260 can employ a nucleotide represented by SEQ ID NO:9 as a forward primer, a nucleotide represented by SEQ ID NO:10 as a reverse primer, a nucleotide represented by SEQ ID NO:11 as a VIC probe, and a nucleotide represented by SEQ ID NO:12 as a FAM probe with the same protocol as in Assays-on-Demand®.

The method for extracting genomic DNA and the method for detecting the base concerned can employ known methods (Bruce, B et al.: Genome Analysis/A laboratory Manual (vol. 4), Cold Spring Harbor Laboratory, NY., 1999, etc.) With regard to the method for detecting the base concerned, there is a method in which a gene sequence in the region concerned is directly determined and, in addition, in the case where a polymorphic sequence is a restriction enzyme recognition site there is a method in which a genotype is determined by utilizing differences in the restriction enzyme cleavage patterns (hereinafter referred as to RFLP) and a method basically employing hybridization using a polymorphism-specific probe (for example, a method in which the polymorphic type is determined by detecting a difference in the extent of hybridization with a specific probe affixed to a chip, a glass slide, or a nylon film, or the genotype is specified by detecting the efficiency of hybridization with a specific probe as an amount of probe that is decomposed by a polymerase when amplifying a template double strand, a method in which a polymorphic type is specified by detecting a difference in temperature of double strand melting while monitoring the temperature change using fluorescence emitted from a certain type of double strand-specific fluorochrome, a method in which a genotype is located by adding complementary sequences to opposite ends of a polymorphic site-specific oligoprobe, and utilizing a difference between formation of a secondary structure within the probe molecule itself and hybridization with a target region by the effect of temperature, etc.) Moreover, there is a method in which a base extension reaction of a template-specific primer is carried out by a polymerase and during this a base that is incorporated into a polymorphic site is located (a method in which dideoxynucleotides are used, they are each fluorescence labeled, and fluorescence from each thereof is detected, a method in which incorporated dideoxynucleotides are detected by mass spectrometry), a method in which, subsequent to a template-specific primer, the presence of a complementary base pair or a noncomplementary base pair in a mutation site is recognized by an enzyme, etc.

These basic detection methods can be carried out, for example, in accordance with known methods such as Bruce, B et al.: Genome Analysis / A laboratory Manual (vol. 4), Cold Spring Harbor Laboratory, NY., 1999 but, with regard to the method for determining a polymorphic genotype, various methods are currently being developed, and it is not limited to those described here.

When employing the above-mentioned methods for directly determining a gene sequence, a polynucleotide coding for an amino acid sequence represented by SEQ ID NO:8, and preferably a polynucleotide formed from a base sequence represented by SEQ ID NO:7, is useful for extracting the gene sequence that is to be determined.

The method for identifying a disease susceptibility gene of the present invention can be implemented by a computer using a program as appropriate.

Such a program typically makes the computer execute
(1) a step in which, based on data for samples from healthy control(s) including base data for a healthy control group, the minor allele frequency is calculated for each SNP, SNPs that have a calculated value of at least a set selection value are selected, and these SNPs are output,
(2) a step in which base data, corresponding to the SNP output in step (1), for a diseased group are input, a comparison is made by statistical processing between the base data for the healthy control group and the base data for the diseased group, and those exhibiting a significant difference are output as chosen SNP markers, and
(3) a step in which base data, corresponding to the SNP markers output in step (2), for a healthy control group and a diseased group that are different from those used in step (2) are input, a comparison is made by statistical processing between the base data for the healthy control group and the base data for the diseased group, and it is determined that a SNP marker that exhibits a significant difference is a disease susceptibility SNP marker, and as a result a method for identifying a disease susceptibility SNP marker is implemented. The program may execute the above-mentioned three steps as a whole.

The program can further include a step in which the disease susceptibility SNP marker is subjected to a linkage disequilibrium analysis so as to locate a region, within a target candidate region, that contains the disease susceptibility SNP marker and where strong linkage disequilibrium is observed. Each of the steps may use a commercial program as appropriate.

Each of the three steps of the program is now explained.
(1) The step in which, based on data for samples from healthy control(s) including base data for a healthy control group, the minor allele frequency is calculated for each SNP, SNPs that have a calculated value of at least a set selection value are selected, and these SNPs are output
   This step implements experimental analysis of samples from healthy control(s), input of experimental information (sample data) including the number of samples used, information about the positions of SNPs on the genome within a candidate region for a disease susceptibility gene, and base information (base data), and selection of SNPs by assessing the input data with reference to a certain level (for example, a gene frequency of at least 10%) of the gene frequency of SNPs (for example, calculated using the number of samples and the base information). In this stage, the positional information is used not to select markers unevenly by choosing those having an appropriately determined certain interval therebetween on the genome and by choosing one from those that are unevenly distributed. The computer stores information on known genomes and known SNPs.
(2) The step in which base data, corresponding to the SNPs output in step (1), for a diseased group are input, a comparison is made by statistical processing between the base data for the healthy control group and the base data for the diseased group, and those exhibiting a significant difference are output as chosen SNP markers
   In this step, when experimental information for the healthy control group including information about the position of SNPs on the genome within the candidate region for the disease susceptibility gene and base information (base data), and experimental information for a diseased group including information about the positions of SNPs on the genome within the candidate region for the disease susceptibility gene and base information (base data) are input, the frequencies observed for the healthy control group and the diseased group are then compared and tested using the input data, and it is judged whether or not the frequency difference is statistically significant. Typically, the presence of an association between a disease and a genetic polymorphism is determined by testing for independence of the genetic polymorphism from the disease by a χ² test. The experimental information input here for the healthy control group can be that input in step (1) or experimental information for a new healthy control group of the present step different from that in step (1).
   Those exhibiting a significant difference between the healthy controls and the diseased individuals are chosen based on the P value, etc. and used as reference markers in the following step.
(3) The step in which base data, corresponding to SNP markers output in step (2), for a healthy control group and a diseased group that are different from those used in step (2) are input, a comparison is made by statistical processing between the base data for the healthy control group and the base data for the diseased group, and it is determined that one that exhibits a significant difference is a disease susceptibility SNP marker

This step is the same as step (2) except that the samples are different.

The statistical processing may be different from the statistical processing used in step (2), and the level of significance employed in this test method can be on a different basis but is preferably statistically more strict than that employed in (2).

The SNP markers that are chosen in step (2) are subjected to further screening and finally specified as a disease susceptibility SNP marker in this step.

The program of the present invention may include, in addition to the above-mentioned three steps, (4) a step in which the disease susceptibility SNP marker is subjected to a linkage disequilibrium analysis and a region, within the target candidate region, where linkage disequilibrium is observed and the disease susceptibility SNP markers is contained is determined.

In this step, the disease susceptibility SNP marker specified in step (3) is subjected to a linkage disequilibrium analysis. From information about the number of samples and the base sequences of healthy controls and diseased individuals, with respect to the extent of linkage disequilibrium between the disease susceptibility SNP marker and SNP markers in the vicinity thereof, a linkage disequilibrium coefficient |D'| between two SNPs is calculated for each of the SNP markers. A certain value of the linkage disequilibrium coefficient |D'| is determined in advance as a reference against which the extent of linkage disequilibrium is evaluated, and by comparing the values calculated above with the reference value a region, within the target candidate region, where strong linkage disequilibrium is observed is located. A gene present in this located region is identified based on known genome information.

Inputting in each step is carried out by a conventional method such as a numeric keypad, a keyboard, a mouse, various types of recording medium, or a network, and outputting is carried out by a conventional method such as displaying, printing out or writing on various types of recording medium.

Furthermore, the method of the present invention can be carried out by a system formed from means for implementing each step as appropriate.

Such a system typically includes
(1) means which calculates the minor allele frequency of each SNP based on data for samples from healthy control(s) including base data for a healthy control group, selects SNPs that have a calculated value of at least a set selection value, and outputs these SNPs,
(2) means into which base data for a diseased group corresponding to the SNPs output in step (1) are input and which compares the base data for the healthy control group with the base data for the diseased group by statistical processing, and outputs as chosen SNP markers those that exhibit a significant difference,
(3) means into which base data corresponding to the SNP markers output in step (2) for a healthy control group and a diseased group that are different from those used in step (2) are input and which compares the base data for the healthy control group with the base data for the diseased group by statistical processing, and determines that one that exhibits a significant difference is a disease susceptibility SNP marker, and
(4) means which subjects the disease susceptibility SNP marker to a linkage disequilibrium analysis and determines a region, within a target candidate region, in which linkage disequilibrium is observed and which contains the disease susceptibility SNP marker, and a disease susceptibility gene can be identified by integrative cooperation of each of the means.

Here, the disease susceptibility gene identification method is implemented not by an integrated computer implementing each step of the program but by the whole system typically using a database on the Internet, etc. Databases that can be used in each step include those used in the above-mentioned identification method.

In the system, means (2) and means (3) employ different samples, but if common statistical processing is employed they can be the same means.

Each of the above-mentioned means is usually formed from a computer, etc. and is equipped with a network interface, a central processing unit (CPU) as control means, a memory such as RAM, a hard disk drive (HDD), a display, an input device including a numeric keypad, a keyboard, a mouse, and an auxiliary recording device, etc.

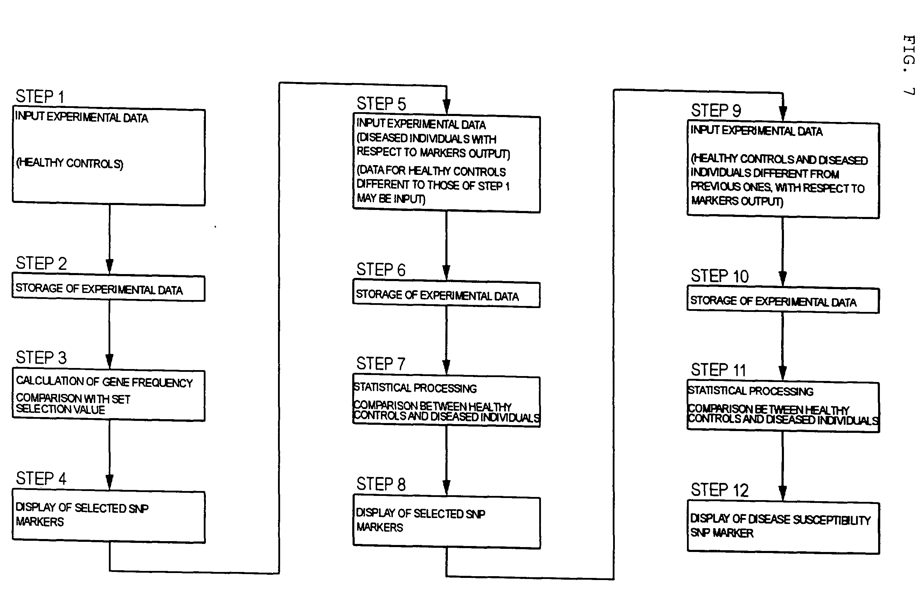

A preferable embodiment of the program of the present invention is explained below by reference to a flowchart of FIG. 7, but it is not limited thereby.

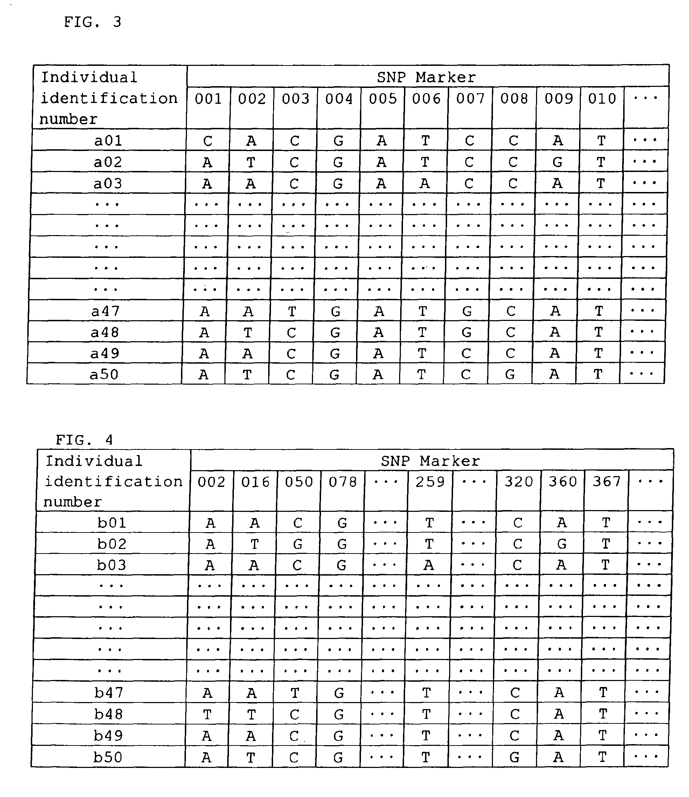

In order to execute the program of the present invention, it is necessary to use a computer equipped with input means, a memory area, processing means, and output (display) means. The memory area stores a table as shown in FIGS. 3 to 6 so that an individual identification number for identifying a sample, a number for identifying a SNP marker, and base data corresponding to a SNP marker obtained for each sample can be input.

### Step 1

A program user inputs, according to the table in FIG. 3, healthy control sample data including base data for a plurality of healthy controls. That is, each healthy control from which a sample is derived is represented by an individual identification number (e.g., a01 to a50), and base data (A, T, C, G, etc.) for numbers corresponding to each of the SNP positions (SNP numbers; e.g., 001, 002, etc. in FIG. 3) are input. Steps 2 to 4

The computer stores these input data in the memory area as in FIG. 3 and uses the data for calculating the gene frequency for each of the SNPs. The computer compares the calculated value with a set value (set selection value) prestored in the memory area, selects SNPs that have a value equal to or greater than the set value, and displays SNP numbers corresponding to these SNPs. Step 5

The user inputs base data obtained from a plurality of diseased individuals (e.g., b01 to b50) corresponding to the SNPs in line with the displayed SNP numbers (e.g., 002, 016, 050, etc. in FIG. 4). Steps 6 to 8

The computer stores these input data in the memory area as in the table of FIG. 4 and uses the data for comparing by statistical processing the base data for the healthy controls with the base data for the diseased individuals. In this stage, the base data for the healthy controls can be the previously input data of FIG. 3 or data obtained separately by experiment in the same manner as in FIG. 4.

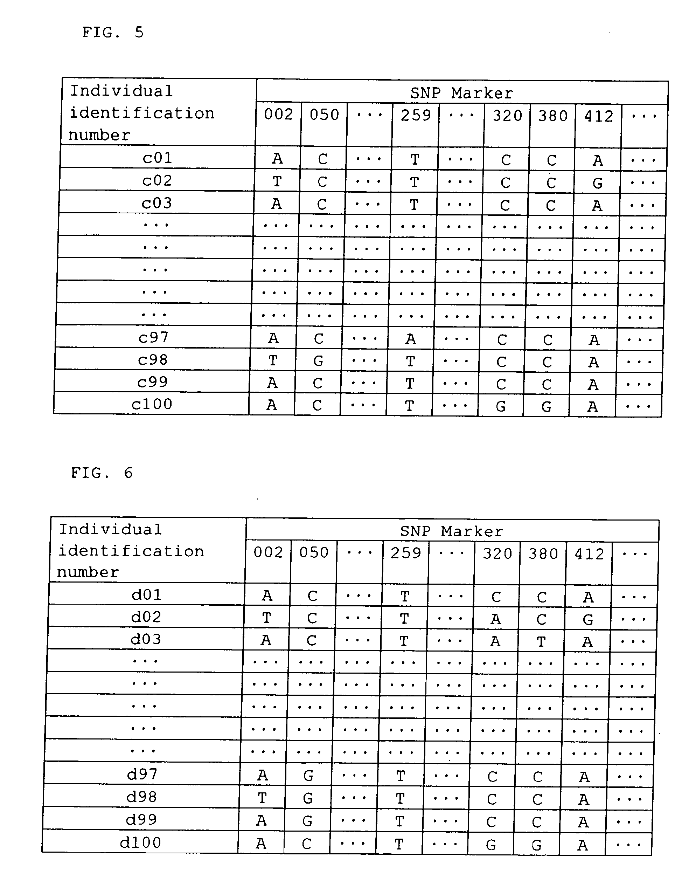

The computer statistically processes the base data for the healthy controls and the base data for the diseased individuals by the processing means and displays those that exhibit a significant difference as chosen SNP markers (e.g., 002, 050, etc. in FIG. 5). Step 9

The user inputs base data for a plurality of healthy controls (e.g., c01 to c100) and base data for a plurality of diseased individuals (e.g., d01 to d100) that are different from those previously used, corresponding to the displayed, chosen SNP markers. Steps 10 to 12

The computer stores these input data in the memory area as in the tables of FIGS. 5 and 6 and uses the data for comparing the base data for the healthy controls with the base data for the diseased individuals by statistical processing.

The base data for the healthy controls and the base data for the diseased individuals are statistically processed by the processing means, and one that exhibits a significant difference is displayed as a disease susceptibility SNP marker.

The present invention is explained in detail below by reference to examples, but the present invention is not limited by these examples.

### Examples

### Example 1 Identification of human type 2 diabetes gene(s)

### 1. Determining target candidate region

Human type 2 diabetes has been intensively studied and, in particular, many reports have been published relating to linkage analysis therefor (Bektas A et al., Diabetes 48(11): 2246-51 (1999); Bektas A et al., Diabetes 50(1):204-8 (2001); Pratley RE et al., J Clin Invest 101(8):1757-64 (1998); Ehm MG et al., Am J Hum Genet 66(6):1871-81 (2000); Wiltshire S et al. Am J Hum Genet 69(3):553-69 (2001)).

From these reports, it can be predicted that human 12th chromosome 12q15-12q22 (a region sandwiched between STS markers D12S375 and D12S362; about 27 Mb) contains a disease susceptibility region that is suggested to have linkage in a plurality of races. This region is therefore determined as a target candidate region for a human type 2 diabetes gene(s). It is conceivable that, since linkage is suggested for a plurality of races in this target candidate region, there is a high possibility that polymorphisms causing development of type 2 diabetes common in the races are present therein.

### 2. Samples

Samples were prepared by collecting peripheral blood from unrelated Japanese type 2 diabetes diseased individuals and unrelated Japanese healthy controls and extracting whole genomic DNA by a standard method.

### 3. SNP typing for unrelated Japanese healthy controls

46 samples from unrelated Japanese healthy controls were subjected to SNP typing with respect to the target candidate region.

SNP typing was carried out by a TaqMan method using, in part, Assays-on-Demand® (manufactured by Applied Biosystems). Furthermore, Dua1384-well GeneAmp® PCR System 9700 (manufactured by Applied Systems) and ABI PRISM® 7900HT Sequence Detection System (manufactured by Applied Systems) instruments were used.

The reaction conditions were as in a manual supplied with the ABI PRISM® 7900HT. That is, the reaction system composition (Table 1) and PCR conditions (Table 2) were as follows.

**Table 1**

| Reaction system composition | |
|---|---|
| Components | Final concentration |
| DNA template | (5 ng) |
| 2 X Universal Master Mix | 1 X |
| Forward primer | 900 nM |
| Reverse primer | 900 nM |
| TaqMan probe (allele 1) | 200 nM |
| TaqMan probe (allele 2) | 200 nM |
| dH₂O | - |

**Table 2**

| PCR conditions | | | |
|---|---|---|---|
| Stage | AmpliTaq Gold Activation | PCR | |
| | Fixed | Cycle (35 cycles) | |
| | | Denature | Anneal/Extension |
| Temperature | 95°C | 92°C | 60°C |
| Period of time | 10 min. | 15 sec. | 1 min. |

As a result of SNP typing, 616 Japanese common SNP markers having a minor allele frequency of at least 15% could be selected within the target candidate region. These 616 SNPs were widely dispersed within the target candidate region, and distributed at a rate of about 1 SNP per 10 kb.

### 4. Evaluation of selected SNP markers

### (A) Evaluation by increasing the number of samples

The above-mentioned SNP typing was carried out for separately prepared samples from 164 unrelated Japanese healthy controls.

Within the target candidate region, 588 SNPs duplicating the previously obtained 616 SNPs were obtained, which had a minor allele frequency of at least 15%.

Although the difference of 28 SNPs might be pseudopositives, this is only 4.5% of 616 SNPs, and 588 SNPs, which corresponds to 95.5%, were obtained, making it clear that the previous SNP typing using 46 cases could give sufficient results.

### (B) Hardy-Weinberg equilibrium test

The 164 unrelated Japanese healthy control samples were used and it was determined that, among the 616 SNPs obtained above, 7 SNPs (1.1% of the total) and 28 SNPs (4.5% of the total) deviated from Hardy-Weinberg equilibrium with statistical significance when the significance levels α were 0.01 and 0.05, respectively.

These results suggested that the deviation from Hardy-Weinberg equilibrium was within the predicted range and the validity of sampling and the validity of SNP typing were confirmed.

### 5. Association analysis (first stage)

An association analysis (first stage: a χ² test for gene frequency) was carried out, with respect to the 616 SNPs obtained above, for 164 samples from unrelated Japanese healthy controls and 164 samples from unrelated Japanese type 2 diabetes diseased individuals.

4 SNPs (0.64% of the total) and 19 SNPs (3.1% of the total) exhibited a statistically significant difference in gene frequency between the healthy controls and the type 2 diabetes diseased individuals when the significance levels α were 0.01 and 0.05, respectively.

Including these SNPs, 40 SNPs (6.5%) that showed a P value of less than 0.10 were chosen as target candidate SNPs for the subsequent association analysis (second stage).

The 40 target candidate SNPs are shown in Table 3.

Typing of each SNP can be carried out by a standard method utilizing Assays-on-Demand® (manufactured by Applied Biosystems), using the dbSNP database, etc. for information on the sequences surrounding the SNPs, and designing primers as appropriate. The product numbers of Assays-on-Demand® utilized in part, and the available dbSNP-ID or the position (position from the end of the short arm of human chromosome 12) on the human genome from the Public Human Genome Draft (June 2002 edition; UCSC Genome Bioinformatics Site, URL http://genome.ucsc.edu/) are given in the Table.

**Table 3**

| Gene frequency, test statistics, and odds ratio of association analysis (first stage) for 40 SNPs that are analysis targets for association analysis (second stage) | | | | | | | |
|---|---|---|---|---|---|---|---|
| Marker | Position in Human genome^{*1)} [bp] | Assays-on-Demand Assay ID | dbSNP ID^{*2)} | Minor allele gene frequency | | χ² test p value | Odds ratio^{*3)} |
| | | | | Control | Diseased | | |
| SNP002 | 68,401,094 | | | 36.8% | 45.7% | 0.0205 | 1.45 |
| SNP017 | 68,946,706 | C 3191343_1_ | rs2293637 | 79.0% | 84.5% | 0.0691 | 1.45 |
| SNP048 | 69,821,833 | C 11686793_1_ | rs710779 | 32.5% | 26.5% | 0.0930 | 1.33 |
| SNP049 | 69,822,338 | C 11686791_1_ | rs497758 | 67.5% | 73.6% | 0.0857 | 1.34 |
| SNP051 | 69,840,596 | C 7559252_1_ | rs710777 | 67.2% | 74.4% | 0.0425 | 1.42 |
| SNP085 | 70,821,461 | C 11688394_1_ | rs2278341 | 17.8% | 25.0% | 0.0246 | 1.54 |
| SNP152 | 72,694,654 | C 3188001_10 | | 52.1% | 60.1% | 0.0408 | 1.38 |
| SNP154 | 72,748,291 | C 11684593_10 | rs1844642 | 53.0% | 59.5% | 0.0959 | 1.30 |
| SNP241 | 78,284,844 | | | 25.2% | 36.0% | 0.0027 | 1.67 |
| SNP245 | 79,160,759 | | | 30.8% | 25.0% | 0.0981 | 1.33 |
| SNP246 | 79,170,150 | C 3087646_10 | rs2030461 | 69.2% | 75.2% | 0.0898 | 1.35 |
| SNP260 | 79,685,306 | | | 38.7% | 45.4% | 0.0836 | 1.32 |
| SNP261 | 79,685,607 | C 36618_10 | | 49.1% | 57.4% | 0.0339 | 1.40 |
| SNP262 | 79,687,681 | C 36615_10 | | 38.7% | 45.1% | 0.0966 | 1.30 |
| SNP274 | 79,796,314 | C 11922042_10 | rs1526963 | 22.6% | 28.7% | 0.0736 | 1.38 |
| SNP308 | 80,918,817 | C 393732_10 | rs1358476 | 43.0% | 50.6% | 0.0504 | 1.36 |
| SNP312 | 81,073,789 | | | 29.6% | 23.8% | 0.0935 | 1.35 |
| SNP318 | 81,198,548 | | | 27.9% | 20.7% | 0.0323 | 1.48 |
| SNP338 | 81,445,670 | | | 58.8% | 65.5% | 0.0765 | 1.33 |
| SNP371 | 81,674,002 | C 223022_10 | rs1522315 | 25.8% | 32.2% | 0.0699 | 1.37 |
| SNP376 | 81,728,482 | C 354316_10 | | 24.8% | 34.5% | 0.0072 | 1.59 |
| SNP377 | 81,729,237 | C 354315_10 | | 81.0% | 74.4% | 0.0430 | 1.47 |
| SNP378 | 81,779,797 | C 354309_10 | | 75.9% | 65.5% | 0.0035 | 1.66 |
| SNP379 | 81,780,624 | | | 12.9% | 18.0% | 0.0709 | 1.48 |
| SNP380 | 81,788,816 | C 249791_10 | rs1922416 | 23.9% | 34.4% | 0.0034 | 1.66 |
| SNP388 | 81,941,723 | | | 76.2% | 70.1% | 0.0763 | 1.37 |
| SNP392 | 85,245,874 | C 3191065_10 | rs1404867 | 22.6% | 29.9% | 0.0331 | 1.46 |
| SNP393 | 85,255,019 | C 2025072_10 | | 22.9% | 29.9% | 0.0416 | 1.44 |
| SNP394 | 85,261,955 | | | 77.3% | 70.1% | 0.0371 | 1.45 |
| SNP396 | 85,308,614 | | | 77.3% | 70.1% | 0.0371 | 1.45 |
| SNP397 | 85,310,974 | C 3191069_10 | | 22.7% | 30.1% | 0.0329 | 1.46 |
| SNP401 | 85,360,949 | C 507897_10 | rs2404772 | 22.7% | 29.8% | 0.0406 | 1.44 |
| SNP402 | -- | C 514715_10 | | 84.3% | 76.5% | 0.0129 | 1.64 |
| SNP440 | -- | C 223925_10 | | 21.6% | 28.4% | 0.0473 | 1.43 |
| SNP453 | 90,728,045 | C 423926_10 | | 88.7% | 84.1% | 0.0872 | 1.48 |
| SNP454 | 90,761,261 | | | 11.3% | 15.9% | 0.0872 | 1.48 |
| SNP487 | 93,293,363 | | | 30.1% | 23.8% | 0.0702 | 1.38 |
| SNP488 | 93,493,600 | C 3188143_10 | | 46.6% | 53.7% | 0.0725 | 1.32 |
| SNP517 | 93,816,142 | C 1972921_10 | rs397135 | 56.4% | 62.8% | 0.0947 | 1.31 |
| SNP570 | 94,801,163 | C 1438058_10 | rs2101236 | 58.2% | 65.2% | 0.0647 | 1.35 |

| | | | | | | | |
|---|---|---|---|---|---|---|---|
| *1) Position on genome: Position of marker polymorphism site by the Public Human Genome Draft, June 2002 edition (UCSC Genome Bioinformatics Site, URL http://genome.ucsc.edu/) | | | | | | | |
| *2) NCBI dbSNP homepage, URL:http://www.ncbi.nlm.nih.gov/SNP/ | | | | | | | |
| *3) Odds ratio: odds ratio of high risk allele to low risk allele | | | | | | | |

### 6. Association analysis (second stage)

For the 40 target candidate SNPs, an association analysis (second stage) was carried out for 262 samples from unrelated Japanese healthy controls and 204 samples from unrelated Japanese type 2 diabetes diseased individuals prepared separately from the samples used in the above-mentioned 5.

Among the 40 SNPs, association with human type 2 diabetes was detected in 3 SNPs (SNP260, SNP262, SNP488) with a significance level α of 0.05 (Tables 4 and 5). These 3 SNPs satisfied the Hardy-Weinberg equilibrium condition and their association with human type 2 diabetes was confirmed.

**Table 4**

| Three SNPs whose association with disease was detected by the association analysis (second stage) | | | | |
|---|---|---|---|---|
| (1) SNP 260 | | | | |
| | Allele 1 | Allele 2 | HWE-P | |
| Healthy control | 42.4% | 57.6% | P = 0.608 | P = 0.0249 |
| Diseased individual | 49.8% | 50.2% | P = 0.233 | OR = 1.35 |

| (2) SNP 262 | | | | |
|---|---|---|---|---|
| | Allele 1 | Allele 2 | HWE-P | |
| Healthy control | 42.3% | 57.7% | P = 0.652 | P = 0.0359 |
| Diseased individual | 49.3% | 50.7% | P = 0.158 | OR = 1.31 |

| (3) SNP 488 | | | | |
|---|---|---|---|---|
| | Allele 1 | Allele 2 | HWE-P | |
| Healthy control | 48.1% | 51.9% | P = 0.561 | P = 0.0149 |
| Diseased individual | 56.1% | 43.9% | P = 0.622 | OR = 1.38 |
| Allele 1: Allele with VIC-labeled probe in TaqMan MGB probe set | | | | |
| Allele 2: Allele with FAM-labeled probe in TaqMan MGB probe set | | | | |
| HWE-P: P value in the Hardy-Weinberg equilibrium test for each group | | | | |
| OR: Odds ratio for high risk allele relative to low risk allele | | | | |

**Table 5**

| Disease susceptibility SNP markers | | |
|---|---|---|
| Marker | Base | |
| | Allele 1 | Allele 2 |
| SNP260 | C | G |
| SNP262 | A | G |
| SNP488 | C | T |

### 7. Linkage disequilibrium analysis

The three disease susceptibility SNP markers (SNP260, SNP262, SNP488) and SNPs in the vicinity thereof were subjected to a linkage disequilibrium analysis.

As analysis target samples, 164 samples from unrelated Japanese healthy controls were used, and as analysis target SNPs, the disease susceptibility SNP markers and SNP markers in the vicinity thereof were used. In the analysis, the SNP disease association analysis software "SNPAlyze ver. 2.1" (manufactured by Dynacom Co., Ltd.) was used, and an analysis was carried out by calculating the linkage disequilibrium coefficient |D'| (pair-wise LD coefficient) between two SNPs by a linkage disequilibrium analysis using the EM method in part.

### (1) Linkage disequilibrium analysis in the vicinity of SNP260 and SNP262

A linkage disequilibrium analysis was carried out using 21 SNPs from SNP247 to SNP267 as the analysis target SNPs. The SNPs used in the analysis are shown in Table 6. The product numbers of Assays-on-Demand® utilized in part, and the available dbSNP-ID or the position (position from the end of the short arm of human chromosome 12) on the human genome from the Public Human Genome Draft (June 2002 edition; UCSC Genome Bioinformatics Site, URL http://genome.ucsc.edu/) are given in the table.

**Table 6**

| SNP247 to SNP267 | | |
|---|---|---|
| Marker | Assays-on-Demand ID^{*1)} | dbSNP ID^{*2)} or position on genome^{*3)} |
| SNP247 | C 3087647_10 | (79,170,468) |
| SNP248 | C 11920917_10 | rs1918189 |
| SNP249 | C 80124_10 | (79,596,151) |
| SNP250 | C 386264_10 | (79,604,122) |
| SNP251 | C 386265_10 | rs1405498 |
| SNP252 | C 111505_10 | rs1245807 |
| SNP253 | C 7474026_10 | rs1245821 |
| SNP254 | | rs1245835 |
| SNP255 | | (79,634,699) |
| SNP256 | | (79,657,804) |
| SNP257 | C 12115914_10 | (79,661,661) |
| SNP258 | | (79,668,079) |
| SNP259 | | rs1526954 |
| SNP260 | | (79, 685, 306) |
| SNP261 | C 36618_10 | (79,685,607) |
| SNP262 | C 36615_10 | (79,687,681) |
| SNP263 | C 7472535_10 | rs1245775 |
| SNP264 | C 7472534_10 | rs1245776 |
| SNP265 | C 9286246_10 | rs2701566 |
| SNP266 | | rs1245770 |
| SNP267 | C 7474009_10 | rs1245769 |

| | | |
|---|---|---|
| ^{*1)} Product number of Assays-on-Demand® | | |
| ^{*2)} dbSNP ID corresponding to each SNP. | | |
| ^{*3)} Based on Public Human Genome Draft (June 2002 edition): shown inside parentheses. | | |

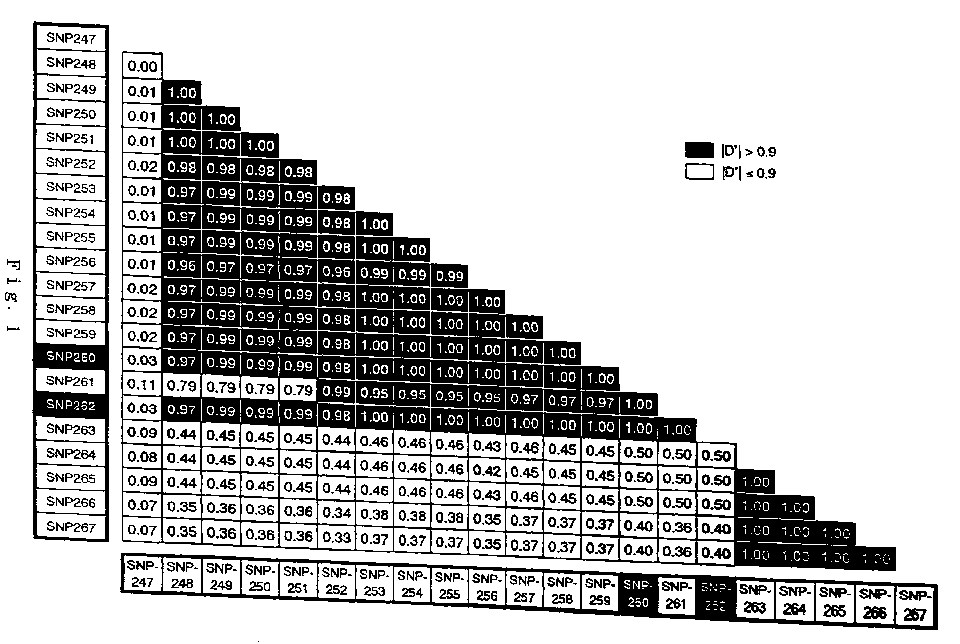

The results are given in FIG. 1. FIG. 1 shows a list of linkage disequilibrium coefficients |D'| between two SNPs and a schematic diagram showing the relative positions of the SNPs.

These results suggest that a haplotype block having very strong linkage disequilibrium showing a |D'|of >0.90 is formed over about 100 kb including from at least SNP248 to SNP262.

It was found by genome analysis that this haplotype block is part of the region of a gene (gene X) genome represented by SEQ ID NO: 7 . As a result, gene X was identified as a human type 2 diabetes susceptibility gene.

### (2) Linkage disequilibrium analysis in the vicinity of SNP488

A linkage disequilibrium analysis was carried out using 21 SNPs from SNP476 to SNP481 and from SNP484 to SNP498 as the analysis target SNPs. The SNPs used in the analysis are shown in Table 7. The product numbers of Assays-on-Demand® utilized in part, and the available dbSNP-ID or the position (position from the end of the short arm of human chromosome 12) on the human genome from the Public Human Genome Draft (June 2002 edition; UCSC Genome Bioinformatics Site, URL http://genome.ucsc.edu/) are shown in the table.

**Table 7**

| SNP476 to SNP481 and SNP484 to SNP498 | | |
|---|---|---|
| Marker | Assays-on-Demand ID^{*1)} | dbSNP ID^{*2)} or position on genome^{*3)} |
| SNP476 | C 1685327_10 | (93,402,266) |
| SNP477 | | (93, 416, 914) |
| SNP478 | | (93,434,423) |
| SNP479 | C 1685357_1_ | rs2291266 |
| SNP480 | | (93,122,256) |
| SNP481 | C 2949342_10 | (93,104,310) |
| SNP484 | C 7625783_10 | rs973601 |
| SNP485 | C 11167851_10 | rs2364227 |
| SNP486 | C 11167872_10 | (93,323,379) |
| SNP487 | | (93,293,363) |
| SNP488 | C 3188143_10 | (93,493,600) |
| SNP489 | | (93,505,991) |
| SNP490 | | (93,509,490) |
| SNP491 | C 3021450_1_ | rs2067016 |
| SNP492 | C 15974673_10 | rs2304439 |
| SNP493 | C 2807903_10 | (93,613,329) |
| SNP494 | C 10079702_10 | (93,617,157) |
| SNP495 | C 2807915_10 | (93,628,532) |
| SNP496 | C 2807918_10 | rs2033669 |
| SNP497 | | (93,636,514) |
| SNP498 | C 3188176_10 | (93,653,222) |

| | | |
|---|---|---|
| *1) Product number of Assays-on-Demand® | | |
| *2) dbSNP ID corresponding to each SNP. | | |
| *3) Based on Public Human Genome Draft (June 2002 edition): shown inside parentheses. | | |

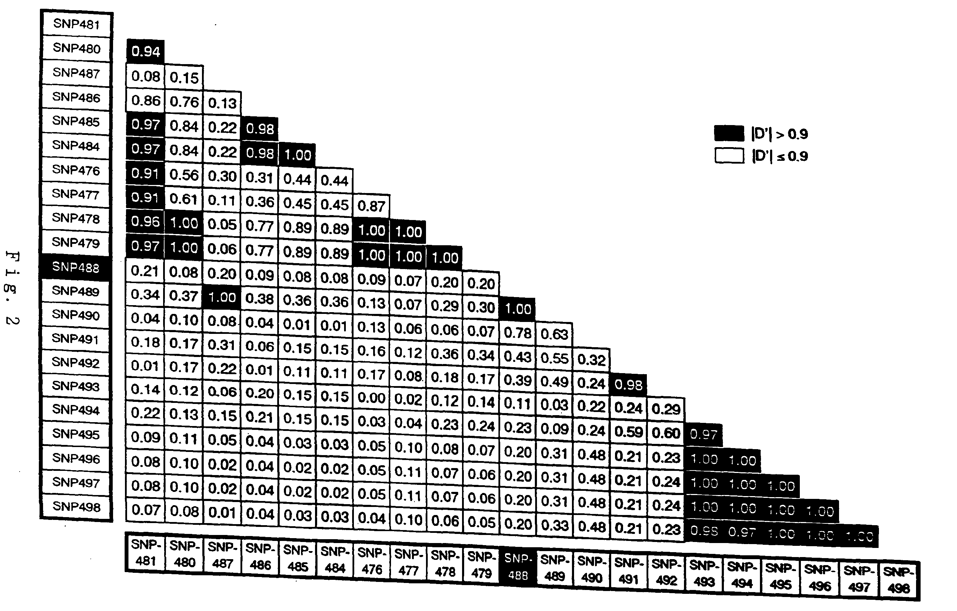

The results are given in FIG. 2. FIG. 2 is a list of linkage disequilibrium coefficients |D'| between two SNPs and a schematic diagram showing the relative positions of the SNPs.

These results suggest that SNP488 is present in a region between a haplotype block of a group before SNP479 and a haplotype block after SNP 491.

It was found by genome analysis that these haplotype blocks correspond to parts of the regions of a gene (gene Y) whose function is unknown. As a result, gene Y was identified as a human type 2 diabetes susceptibility gene.

In accordance with the present example, the above-mentioned two genes were identified as human type 2 diabetes susceptibility genes, the target candidate region (human 12th chromosome 12q15-12q22 (a region sandwiched between STS markers D12S375 and D12S362; about 27 Mb)) is presumed to contain about 170 genes and, among them, two disease susceptibility genes could be efficiently identified, which is highly significant.

### Example 2 Analysis of disease susceptibility gene X

It was found that disease susceptibility gene X identified in Example 1 is the SYT1 gene (NCBI Accession No.: MM_005639). The cDNA base sequence of gene X and the amino acid sequence encoded thereby are shown in SEQ ID NO:7 and SEQ ID NO:8, respectively.

The SYT1 (synaptotagmin 1) gene codes for a membrane protein expressed in a synaptic vesicle or a chromaffin granule within a cell and controls exocytosis and endocytosis. It is conceivable that this functions as a calcium sensor and controls, according to the calcium concentration, transport of the synaptic vesicle and exocytosis of a neurotransmitter within the synaptic vesicle. 13 genes have so far been reported for synaptotagmin; with regard to the SYT3 (synaptotagmin 3) gene, it is reported that it is expressed in pancreatic β cells and relates to exocytosis of insulin, but it has been thought that STY1 is not expressed in pancreatic β cells (Diabetes, 49(3):383-91, 2000.05. Proceedings of the National Academy of Sciences of the United States of America, 91(26):12487-91, 1994.12).

It has been difficult to specify which thereof are human type 2 diabetes susceptibility genes, but in accordance with the method of Example 1, it becomes possible to specify disease susceptibility genes among the large number of genes.

### [Industrial Applicability]

In accordance with the present invention, it becomes possible to select highly reliable SNP markers within a target candidate region where a disease susceptibility gene is thought to be present, the markers being evenly distributed throughout the region at reasonable intervals. In particular, since the markers are selected so as to be evenly distributed throughout a region at reasonable intervals and an association analysis is carried out while dividing a sample panel set into two stages, it has been demonstrated to be extremely effective for the Positional Approach method in which a disease susceptibility gene is identified utilizing linkage disequilibrium.

Furthermore, in accordance with this method, disease susceptibility genes for type 2 diabetes in Japanese people have been successfully identified by carrying out an exhaustive association analysis for a disease susceptibility region which is observed for a plurality of races in common.

## Claims

1. A method for identifying disease susceptibility genes using SNP markers, the method comprising:
(1) a step in which a plurality of SNP markers are selected from within a candidate region for the disease susceptibility gene using samples from healthy control(s), the SNP markers not being unevenly distributed throughout the candidate region;
(2) a step in which, for the SNP markers selected in step (1), a comparison is made by statistical processing between a healthy control group and a diseased group, and SNP markers that exhibit a significant difference are chosen;
(3) a step in which, for the SNP markers chosen in step (2), a comparison is made by statistical processing between a healthy control group and a diseased group that are different from those of step (2), and a SNP marker that exhibits a significant difference is specified as a disease susceptibility SNP marker; and
(4) a step in which a gene is identified by subjecting the disease susceptibility SNP marker to a linkage disequilibrium analysis and locating a region, within the target candidate region, in which linkage disequilibrium is observed and which contains the disease susceptibility SNP marker.

2. The method according to Claim 1, comprising carrying out the selection of SNP markers in step (1) so that the marker density is at least 1 SNP per 10 kb in the target candidate region.

3. The method according to Claim 1 or 2, comprising carrying out the selection of SNP markers in step (1) so that the interval between adjacent SNP markers is at least 5 kb.

4. The method according to any one of Claims 1 to 3, comprising carrying out the selection of SNP markers in step (1) on the basis of gene frequency.

5. The method according to Claim 4 wherein the basis of gene frequency is that the minor allele gene frequency is at least 10%.

6. The method according to Claim 4 wherein the basis of gene frequency is that the minor allele gene frequency is at least 15%.

7. The method according to any one of Claims 1 to 6, comprising evaluating the selected SNP markers by repeating step (1) using samples from healthy control(s) that are different from those used in step (1).

8. The method according to Claim 7 wherein the evaluation is carried out by a Hardy-Weinberg equilibrium test.

9. The method according to any one of Claims 1 to 8 wherein the statistical processing in step (2) is an association analysis.

10. The method according to any one of Claims 1 to 9 wherein the statistical processing in step (3) is an association analysis.

11. The method according to any one of Claims 1 to 10 wherein the significance level in the comparison in step (3) is lower than the significance level in the comparison in step (2).

12. The method according to Claim 11 wherein the association analysis in step (2) is carried out by a χ² test for gene frequency, and a SNP marker that exhibits a significant difference with a significance level of α ≤ 0.10 is chosen, and the association analysis in step (3) is carried out by a χ² test for gene frequency, and a SNP marker that exhibits a significant difference with a significance level of α < 0.10 is chosen.

13. The method according to any one of Claims 1 to 12 wherein the linkage disequilibrium analysis for the disease susceptibility SNP marker in step (4) is carried out for the SNP markers that are selected in step (1) and the disease susceptibility SNP marker.

14. The method according to any one of Claims 1 to 12 wherein the number of SNP markers that are subjected to the linkage disequilibrium analysis, including the disease susceptibility SNP marker, is at least 4.

15. A disease susceptibility marker that is specified by means of the ability to exhibit a significant difference when comparing by statistical processing, using samples from healthy control(s), a healthy control group and a diseased group with respect to a plurality of SNP markers that are selected so as not to be unevenly distributed throughout a candidate region for a disease susceptibility gene, choosing SNP markers that exhibit a significant difference, and further comparing by statistical processing a different healthy control group and a different diseased group with respect to the chosen SNP markers.

16. A disease diagnosis marker containing one or more polynucleotides chosen from the group consisting of human genome polynucleotides having a length that can be specifically recognized on the human genome, the polynucleotides containing each SNP marker among one or more SNP markers that are present in a region where linkage disequilibrium is observed within a target candidate region in a linkage disequilibrium analysis with respect to the disease susceptibility marker according to Claim 15, the region where linkage disequilibrium is observed containing the disease susceptibility SNP marker.

17. A diabetes susceptibility diagnosis marker containing one or more polynucleotides chosen from the group consisting of
a polynucleotide in which a base sandwiched between a sequence represented by SEQ ID NO:1 and a sequence represented by SEQ ID NO:2 within a genomic sequence is C or G,
a polynucleotide in which a base sandwiched between a sequence represented by SEQ ID NO:3 and a sequence represented by SEQ ID NO:4 within the genomic sequence is A or G, and
a polynucleotide in which a base sandwiched between a sequence represented by SEQ ID NO:5 and a sequence represented by SEQ ID NO:6 within the genomic sequence is C or T.

18. A diabetes susceptibility diagnosis method comprising:
(1) a step in which genomic DNA is extracted from a sample, and
(2) a step including, with regard the sequence of the extracted genomic DNA, one or more chosen from the group consisting of
detecting a base sandwiched between a sequence represented by SEQ ID NO:1 and a sequence represented by SEQ ID NO:2,
detecting a base sandwiched between a sequence represented by SEQ ID NO: 3 and a sequence represented by SEQ ID NO: 4, and
detecting a base sandwiched between a sequence represented by SEQ ID NO:5 and a sequence represented by SEQ ID NO:6.

19. A program that allows a computer to execute
(1) a step in which, based on data for samples from healthy control(s) including base data for a healthy control group, the minor allele gene frequency is calculated for each SNP, SNPs that have a calculated value of at least a set selection value are selected, and these SNPs are output,
(2) a step in which base data, corresponding to the SNPs output in step (1), for a diseased group are input, a comparison is made by statistical processing between the base data for the healthy control group and the base data for the diseased group, and SNPs that exhibit a significant difference are output as chosen SNP markers, and
(3) a step in which base data, corresponding to the SNP markers output in step (2), for a healthy control group and a diseased group that are different from those used in step (2) are input, a comparison is made by statistical processing between the base data for the healthy control group and the base data for the diseased group, and it is determined that a SNP marker that exhibits a significant difference is a disease susceptibility SNP marker.

20. A disease susceptibility gene identification system for identifying a disease susceptibility gene, the system comprising:
(1) means for calculating, based on data for samples from healthy control(s) including base data for a healthy control group, the minor allele frequency for each SNP, selecting SNPs that have a calculated value of at least a set selection value, and outputting these SNPs,
(2) means for inputting base data for a diseased group corresponding to the SNPs output by means (1), comparing by statistical processing the base data for the healthy control group and the base data for the diseased group, and outputting those that exhibit a significant difference as chosen SNP markers,
(3) means for inputting base data, corresponding to the SNP markers output by means (2), of a healthy control group and a diseased group that are different from those used for means (2), comparing by statistical processing the base data for the healthy control group and the base data for the diseased group, and determining that one that exhibits a significant difference is a disease susceptibility SNP marker, and
(4) means for subjecting the disease susceptibility SNP marker to a linkage disequilibrium analysis and determining within a target candidate region a region in which linkage disequilibrium can be observed and which contains the disease susceptibility SNP marker.
